Europäisches Patentamt

European Patent Office

Office européen des brevets

(19)

(11) Veröffentlichungsnummer : **0 366 015 B1**

(12) **EUROPÄISCHE PATENTSCHRIFT**

(45) Veröffentlichungstag der Patentschrift :
**10.03.93 Patentblatt 93/10**

(51) Int. Cl.$^5$ : **C07C 303/24**

(21) Anmeldenummer : **89119501.8**

(22) Anmeldetag : **20.10.89**

(54) **Verfahren zur Herstellung hochsulfatierter Fettsäuren und Fettsäurederivate.**

Verbunden mit 89912428.3/0440730
(europäische
Anmeldenummer/Veröffentlichungsnummer)
durch Entscheidung vom 01.06.92.

(30) Priorität : **26.10.88 DE 3836447**

(43) Veröffentlichungstag der Anmeldung :
**02.05.90 Patentblatt 90/18**

(45) Bekanntmachung des Hinweises auf die
Patenterteilung :
**10.03.93 Patentblatt 93/10**

(84) Benannte Vertragsstaaten :
**AT BE CH DE ES FR GB GR IT LI LU NL SE**

(56) Entgegenhaltungen :
**DE-C- 561 715**

(73) Patentinhaber : **Chemische Fabrik
Stockhausen GmbH
Bäkerpfad 25
W-4150 Krefeld (DE)**

(72) Erfinder : **Peppmöller, Reinmar,
Dr.-Dipl.-Chem.
Kemmerhofstrasse 189
W-4150 Krefeld (DE)**
Erfinder : **Dahmen, Kurt, Dr.-Dipl.-Chem.
Von-Velsen-Strasse 6
W-4050 Mönchengladbach-Rheydt (DE)**

(74) Vertreter : **Klöpsch, Gerald, Dr.-Ing.
Patentanwalt
An Gross St. Martin 6
W-5000 Köln 1 (DE)**

EP 0 366 015 B1

## Beschreibung

Die Erfindung betrifft ein Verfahren zur Herstellung von hochsulfatierten Fettsäuren, Hydroxifettsäuren und/oder oxalkylierter Hydroxifettsäuren.

Die Umsetzung von Fettsäuren und Fettsäureestern mit Schwefelsäure oder Oleum zur Herstellung von wasserlöslichen bzw. mit Wasser mischbaren Produkten mit oberflächenaktiven Eigenschaften hat eine breite industrielle Anwendung gefunden. Ausgehend von beispielsweise Rizinusöl, Olivenöl, Sojabohnenöl, Sonnenblumenöl, Talg oder verschiedenen Fischölen, sowie den darin gebundenen, durch Verseifung erhältlichen natürlichen Fettsäuren konnten abhängig von bestimmten Reaktionsbedingungen sogenannte "sulfatierte Öle" für die unterschiedlichsten Anwendungszwecke erzeugt werden.

Ein besonders bekannter Vertreter dieser Stoffklasse ist sulfatiertes Rizinusöl, welches bis heute unter der Bezeichnung "Türkischrotöl" im Handel ist.

Nach einem in dem DRP 561 715 beschriebenen Verfahren gelingt es, durch Kurzzeitsulfatierung mit großen Schwefelsäuremengen unter intensiver Kühlung Produkte mit außerordentlicher Säure- und Salzbeständigkeit herzustellen. Die analytischen Untersuchungen ergaben, daß diese außergewöhnlichen Netzmittel einen wesentlich höheren Gehalt an organisch gebundenem Schwefel aufweisen, als mit anderen Verfahren erhältlich ist.

Diesen Ergebnissen entsprechend gingen die Bemühungen dahin, einen möglichst hohen Sulfatierungsgrad zu erreichen. Der Sulfatierungsgrad gibt an, wieviel Prozent der im untersuchten Produkt enthaltenen Fettsäuren tatsächlich sulfatiert sind, wobei willkürlich angenommen wird, daß die gesamte organisch gebundene Schwefelsäure (berechnet als $SO_3$) in Form von Rizinolsäure-Monoschwefelsäureester vorliegt (H.P. Kaufmann, Analyse der Fette und Fettprodukte, Springer-Verlag 1958, Seite 1495). Wegen der anwendungstechnischen Eigenschaften war und ist insbesondere ein Sulfatierungsgrad von mindestens 50 % erwünscht, in diesem Sinn wird im folgenden die Bezeichnung "hochsulfatiert" bzw. "hoher Sulfatierungsgrad" verwendet.

Eine Steigerung des Sulfatierungsgrades auf Werte über 50 % setzt nach bekannten Verfahren die Verwendung von Lösungsmitteln, beispielsweise Trichlorethylen, voraus. Hierbei dient das Lösungsmittel einerseits als Verdünnungsmittel während der Sulfierung und andererseits als Mittel zur Abtrennung der nicht sulfierten Anteile aus dem Reaktionsprodukt.

Eine Möglichkeit, nicht sulfierte Anteile ohne Verwendung eines Lösungsmittels aus einem Sulfierungsgemisch abzutrennen, ist in der DE-OS 36 06 868 beschrieben. Nach dieser Veröffentlichung werden bei der Herstellung ungesättigter Fettsäureniedrigalkylester-Sulfonate artfremde Substanzen mit Demulgatorwirkung zugesetzt. Empfohlen werden pro 100 Gew.-% sulfoniertem Ester Beigaben von 5 bis 400 Gew.-Teilen Sorbitan- oder Glycerinfettsäureester sowie 5 bis 300 Gew.-Teile eines niedrigmolekularen Alkohols.

Der vorliegenden Erfindung liegt somit die Aufgabe zugrunde, ein Verfahren zur Herstellung hochsulfatierter Fettsäuren, Hydroxifettsäuren und/oder oxalkylierter Hydroxifettsäuren bereitzustellen, welches einerseits ermöglicht, den Einsatz von Lösungsmitteln, die damit verbundenen Toxizitätsprobleme und den apparativen Aufwand zu vermeiden und welches anderer seits ohne den Zusatz artfremder Stoffe auskommt, die die anwendungstechnischen Eigenschaften der Endprodukte beeinträchtigen können.

Diese Aufgabe wird durch das Verfahren gemäß der Erfindung gelöst, bei dem nach Durchführung der an sich bekannten Sulfatierung das Reaktionsgemisch, welches neben den sulfatierten Estern auch unsulfatierte Ausgangsprodukte enthält, neutralisiert wird und gegebenenfalls nach Abtrennung der Salzsole das neutrale Estersulfat mit Wasser verdünnt wird. Nach Zugabe einer dem organisch gebundenen Schwefelsäuregehalt äqui- oder submolaren Laugenmenge wird bei Temperaturen zwischen 20 °C und 160°C, vorzugsweise zwischen 40 und 90°C die erhaltene alkalische Lösung hydrolysiert, wobei sich eine aus unsulfatierten Estern bestehende, wasserunlösliche Ölschicht von der wässrigen Phase, die die erwünschten sulfatierten Fettsäuren enthält und aus der diese anschließend isoliert werden können, abscheidet.

Vorzugsweise wird das aus der Sulfatierungsreaktion erhaltene, normalerweise neutral eingestellte Estersulfat mit mindestens der halben Gewichtsmenge, weiter bevorzugt der doppelten bis dreifachen Gewichtsmenge Wasser, bezogen auf das Gewicht des Estersulfatgemischs, verdünnt.

Vorteilhaft ist ein Wassergehalt der alkalischen Mischung zu Beginn der Hydrolyse von 60 bis 95 Gew.-%, vorzugsweise 70 bis 90 Gew.-%, bezogen auf die Gesamtmischung.

Erfindungsgemäß kann das gegebenenfalls entsalzte Estersulfatgemisch mit 10 Gew.-% bis 50 Gew.-% eines bereits hydrolysierten Estersulfats, bezogen auf das nichthydrolysierte Estersulfat, vermischt und anschließend der weiteren Hydrolyse bei Temperaturen von mindestens 20°C unterworfen werden. Die Hydrolyse kann drucklos oder unter Druck erfolgen.

Geeignete Ausgangsprodukte sind ungesättigte Fettsäureester, gesättigte oder ungesättigte Hydroxifettsäureester oder die Alkoxylierungsprodukte gesättigter oder ungesättigter Hydroxifettsäureester von wasserlöslichen ein- oder zweiwertigen Alkoholen, wie Methanol, Ethanol, n-Propanol, i-Propanol, Ethylenglykol, 1,2-Propylenglykol, 1,3-Propylenglykol, 1,4-Butylenglykol und Hexylenglykol.

Leicht zugängliche Fettsäureester wie Ölsäuremethylester, Tallölfettsäuremethylester, Fischölfettsäuremethylester, Rapsölfettsäuremethylester, Ölsäureethylester, Ölsäureisopropylester, Ethylenglykoldioleat, 1,2-Propylenglykoldioleat, Rizinusfettsäuremethylester, Rizinusfettsäureethylester und die alkoxylierten Derivate der Rizinusfettsäureester, soweit sie wasserunlöslich sind, sind bevorzugt.

Die genannten Ausgangsprodukte können allein oder im Gemisch eingesetzt werden und sind vorzugsweise im wesentlichen frei von Fettsäuren.

Je nach Provenienz enthalten die Ausgangsester Anteile von nichtsulfatierbaren gesättigten Estern, die das erfindungsgemäße Verfahren jedoch nicht stören und die zusammen mit den nicht sulfatierten ungesättigten Estern von den erwünschten sulfatierten Produkten abgetrennt werden können.

Freie Fettsäuren und Seifen können jedoch das erfindungsgemäße Herstellungsverfahren beeinträchtigen. Daher sollten dann, wenn sehr hohe Trennleistungen gefordert werden, bevorzugt Fettsäureester mit geringstmöglicher Aufspaltung eingesetzt werden.

Bei der Umsetzung dieser Ester mit Schwefelsäure oder Oleum zu den Estersulfaten, die nach üblichen Methoden erfolgen kann, spielt der erreichte Sulfatierungsgrad keine entscheidende Rolle.

Der erfindungsgemäße Verdünnungsschritt führt unter Zugabe von Basen zur Phasentrennung in eine obere, ölige Phase und eine untere, wäßrige, die gewünschten sulfatierten Fettsäuren enthaltende Phase. Die obere Ölphase, die auch als "Neutralöl" bezeichnet wird, enthält im wesentlichen unsulfatierte Ester.

Zu der alkalischen Verseifung kann jede zur Fettspaltung geeignete Lauge verwendet werden, insbesondere starke Laugen wie Natronlauge und Kalilauge. Die leichte Verseifbarkeit der Fettsäureestersulfate ermöglicht jedoch auch den Einsatz schwächerer Basen wie Ammoniak oder Amine.

Die Laugenmenge richtet sich nach dem gewünschten Anteil sulfatierter Seife sowie Alkohol in der Estersulfatlösung und kann zwischen einer 10- und 100 %igen Aufspaltung des Estersulfats frei gewählt werden.

Die Alkalibehandlung in Gegenwart größerer Wassermengen gemäß der Erfindung führt praktisch ausschließlich zu einer Verseifung des Fettsäureestersulfats.

Eine Hydrolyse der unsulfatierten Anteile des Gemischs findet höchstens in einem zu vernachlässigenden Ausmaß statt.

Die alkalische Lösung wird so lange auf eine Temperatur zwischen 20°C und 160°C, bevorzugt zwischen 40 und 90°C, erhitzt, bis die Lauge verbraucht ist und die unsulfatierten Anteile sich in Form der oberen Neutralölschicht abscheiden.

Eine größere als dem organisch gebundenen Schwefelsäuregehalt äquivalente Laugenmenge ist zu vermeiden, da bei Laugenüberschuß auch der unsulfatierte Ester gespalten würde. Diese unerwünschte Verseifung kann durch Zusatz von unterstöchiometrischen Laugenmengen vermieden werden. Wenn das aus der Sulfatierungsreaktion gewonnene Estersulfatgemisch zwischengelagert werden muß, kann es von Vorteil sein, bei der Neutralstellung des sauren Estersulfats durch einen geringen Überschuß von Lauge eine alkalische Stabilisierung vorzunehmen, um eine unkontrollierte saure Aufspaltung zu vermeiden. Dieser Laugenüberschuß muß dann bei der späteren Laugenzugabe berücksichtigt werden, so daß die zugesetzte Gesamtmenge dem organisch gebundenen Schwefelsäuregehalt höchstens äquivalent ist.

Während der Verseifungsreaktion wird die je nach Sulfatierungsgrad klare oder milchige wässrige Lösung des Sulfatierungsgemischs zunehmend trüber und es scheidet sich schließlich Öl ab. Die Phasentrennung kann physikalisch beschleunigt werden, z.B. mittels Zentrifugen oder Fullkörperkolonnen.

Die Zeit bis zum Beginn der Ölabscheidung hängt weitgehend von der Art des Fettsäureesters, dem anorganischen Salzgehalt und der Temperatur der Behandlung ab. Während sulfatierte Ester der primären Alkohole bei dem erfindungsgemäßen Verfahren schnell die unsulfatierten Anteile abscheiden, erfolgt die Abscheidung bei den sulfatierten Estern zweiwertiger Alkohole langsamer; die letzteren neigen auch stärker zu einer bleibenden Aufnahme der Neutralölanteile. Die Verfahrensoptimierung erfolgt je nach Zielvorgabe im Einzelfall durch Variation von Temperatur und - über die Laugenmenge - des Verseifungsgrades.

Es ist jedoch auch möglich und bisweilen empfehlenswert, einen nur träge reagierenden sulfatierten Ester mit einem schnellreagierenden Ester, z.B. einem sulfatierten Methylester, zu vermischen. Der zusätzlich eingesetzte sulfatierte Ester kann bezüglich der Fettsäurebasis und/oder des Sulfatierunggrades sowohl gleich als auch ungleich sein.

Die Mitverwendung des im Hinblick auf die nachfolgende Hydrolyse und Phasentrennung geeigneten Zusatzesters direkt bei der Sulfatierungsreaktion, so daß bereits ein Gemisch verschiedener geeigneter Ester gemeinsam sulfatiert und anschließend den weiteren erfindungsgemäßen Schritten unterworfen wird, ist vorteilhaft.

Das erfindungsgemäße Verfahren kann in den einzelnen Stufen sowohl diskontinuierlich als auch kontinuierlich durchgeführt werden. Bei Kurzzeit-Erhitzung der alkalischen Estersulfatlösung unter Druck, beispielsweise innerhalb eines Röhren- oder Fallfilmreaktors, ist die erforderliche Verseifungstemperatur der Reaktorgeometrie und der Durchflußgeschwindigkeit anzupassen. Hierbei gilt die Regel, daß eine Verkürzung der Verweilzeit der Estersulfatlösung in der Heizstrecke eine Erhöhung der Verseifungstemperatur erfordert.

Das erfindungsgemäße Verfahren wird anhand der nachfolgenden Beispiele, die in Form allgemeiner Arbeitsvorschriften I bis IV mit den zugehörigen Tabellen dargestellt sind, erläutert.

## Allgemeine Arbeitsvorschrift I

A g eines mit konzentrierter Schwefelsäure hergestellten und mit Natronlauge neutralisierten Estersulfates bzw. Estersulfatgemisches, welches bei einem $SO_3$-Gehalt von B % einen Sulfatierungsgrad $S_1$ aufwies, wurden mit 2.000 ml enthärtetem Wasser verdünnt. Danach wurde eine dem $SO_3$-Gehalt äquimolare Menge 45%ige Natronlauge zugesetzt (Menge C). Nach kräftiger Durchmischung wurde die alkalische Lösung auf ca. 50 °C erhitzt, wobei starke Eintrübung und schließlich Bildung von Schichten erfolgte. Die Phasentrennung ergab D g unsulfatierten Fettsäureester.

Aus der unteren, wäßrigen Phase wurde nach Zusatz von Mineralsäure (pH= 4) und Kochsalz das sulfatierte Fettsäuresulfat erhalten. Die Sulfatierungsgrade der Ausgangs- und Endprodukte ($S_1$ und $S_2$) sind der Tabelle I zu entnehmen.

Die Hydrolyse bei den Beispielen h und i wurde mittels 45 %iger Kalilauge bzw. konzentrierter Ammoniaklösung durchgeführt.

## Allgemeine Arbeitsvorschrift II

A g eines mit konzentrierter Schwefelsäure hergestellten und mit Natronlauge neutralisierten Estersulfates bzw. Estersulfatgemisches, das bei einem $SO_3$-Gehalt von B %einen Sulfatierungsgrad $S_1$ aufwies, wurden mit 2.000 ml enthärtetem Wasser verdünnt. Danach wurde eine auf den $SO_3$–Gehalt bezogene submolare Menge 45 %ige Natronlauge (Menge C) zugesetzt. Die weitere Aufarbeitung erfolgte wie unter I.

Die Versuchsergebnisses sind in Tabelle II zusammengefaßt.

## Allgemeine Arbeitsvorschrift III

A g eines mit konzentrierter Schwefelsäure hergestellten und mit Natronlauge neutralisierten Estersulfats bzw. Estersulfatgemischs, das bei einem $SO_3$-Gehalt von B %einen Sulfatierungsgrad $S_1$ aufwies, wurden mit 400 ml enthärteten kochendem Wasser verdünnt. Anschließend wurde die Mischung auf 80°C gebracht und eine auf den $SO_3$-Gehalt bezogene äquimolare Menge 45 %ige Natronlauge zugesetzt (Menge C). Nach kräftiger Durchmischung wurde die alkalische, trübe Flüssigkeit in eine Zentrifuge gegeben und 3 Minuten bei 3000 Upm zentrifugiert (Martin Christ, Osterode; Typ UJ 3, 330 Watt). Die weitere Aufarbeitung erfolgte wie unter I.

Die Ergebnisse sind in Tabelle III dargestellt.

Die Herstellung des den allgemeinen Arbeitsvorschriften I, II und III zugrundeliegenden Ausgangsmaterials wird beispielhaft anhand des Ölsäuremethylesters beschrieben:

538 g Ölsäuremethylester (Edenor ME TI 05 der Fa. HENKEL) wurden mit 350 g konz. Schwefelsäure unter Eiskühlung (max. 10°C) bei intensivem Rühren innerhalb 1 Stunde sulfatiert. Danach erfolgte zur Neutralisation auf einen pH-Wert von 5 bis 6 und Aufarbeitung die Zugabe von 1676 g einer alkalisch-wäßrigen Lösung, die 225 g Ätznatron und 120 g Kochsalz enthielt, so schnell wie möglich. Die Temperatur stieg dabei auf ca. 80°C. Nach dem Stehenlassen der Mischung bei 50 bis 60°C wurde die Salzlösung abgezogen und das erhaltene Estersulfat hinsichtlich Wassergehalt und Sulfatierungsgrad bestimmt (Ausbeute: 1000 g).

```
Wassergehalt :                        40 %

Gehalt an wirksamer Substanz:  60 %

Sulfatierungsgrad:              ca. 50 %
```

Dieses Produkt wurde den Verfahren entsprechend den allge- meinen Arbeitsvorschriften I (Beispiele h, i), II (Beispiele b,c,d) und III (Beispiel a) unterworfen, wobei unsulfatierter Methylester aus der wäßrigen Phase ausgeschieden wurde und der Sulfatierungsgrad entsprechend anstieg.

EP 0 366 015 B1

Allgemeine Arbeitsvorschrift IV

Nach der allgemeinen Arbeitsvorschrift I werden A g eines mit konz. Schwefelsäure hergestellten und mit Natronlauge neutralisierten Estersulfats mit einem $SO_3$-Gehalt von B % und einem Sulfatierungsgrad $S_1$ % mit 500 ml g enthärtetem Wasser verdünnt. Hydrolyse und weitere Aufarbeitung erfolgte nach der allgemeinen Arbeitsvorschrift I.

Die Ergebnisse sind in Tabelle IV dargestellt.

Tabelle I                    Allgemeine Arbeitsvorschrift I

| | Einsatz (g) A | H$_2$O-Zusatz (g) | Verhältnis A:H$_2$O | H$_2$O-Gehalt (%) * | SO$_3$ (%) B | NaOH 45% (g) C | Unsulf. (g) D | S$_1$ (%) | S$_2$ (%) |
|---|---|---|---|---|---|---|---|---|---|
| a) Ölsäuremethylester | 1000 | 2000 | 1:2,0 | 80,0 | 8,2 | 90 | 201 | 57 | 87 |
| b) Tallölfettsäuremethylester | 1134 | 2000 | 1:1,76 | 78,3 | 8,6 | 108 | 182 | 58 | 82 |
| c) Ölsäureisopropylester | 796 | 2000 | 1:2,51 | 82,9 | 9,5 | 82 | 151 | 56 | 83 |
| d) 1,2-Propylenglykol-dioleat | 984 | 2000 | 1:2,03 | 80,2 | 7,7 | 84 | 106 | 48 | 61 |
| e) Rizinolsäuremethylester | 1346 | 2000 | 1:1,49 | 75,8 | 7,1 | 106 | 470 | 39 | 71 |
| f) Oxethylierter Rizinolsäuremethylester (3 Mole EO) | 984 | 2000 | 1:2,03 | 80,2 | 6,1 | 66 | 130 | 41 | 76 |
| g) Ölsäuremethylester Ölsäureisopropylester (1:1 gemischt) | 1000 | 2000 | 1:2,00 | 80,0 | 8,4 | 93 | 193 | 57 | 85 |
| h) Ölsäuremethylester | 1500 | 2000 | 1:1,33 | 74,3 | 4,8 | KOH 45% 112 | 145 | 50 | 89 |
| i) Ölsäuremethylester | 1500 | 2000 | 1:1,33 | 74,3 | 4,8 | NH$_3$conc. 106 | 65 | 50 | 68 |

* H$_2$O-Gehalt vor Hydrolyse bezogen auf einen durchschnittlichen Wassergehalt des Ausgangsprodukts von 40 %.

Tabelle II

Allgemeine Arbeitsvorschrift II

| | Einsatz (g) $\underline{A}$ | $H_2O$- Zusatz (g) | Verhältnis A : $H_2O$ | $H_2O$- Gehalt (%) * | $SO_3$ (%) $\underline{B}$ | NaOH,45% (g) $\underline{C}$ | Unsulf. (g) $\underline{D}$ | $S_1$ (%) | $S_2$ (%) |
|---|---|---|---|---|---|---|---|---|---|
| a) Ölsäuremethylester | 750 | 2000 | 1:2,67 | 83,6 | 8,2 | 41 | 229 | 43 | 81 |
| b) " | 1000 | 2000 | 1:2,00 | 80,0 | 4,8 | 21 | 79 | 50 | 80 |
| c) " | 1000 | 2000 | 1:2,00 | 80,0 | 4,8 | 16 | 68 | 50 | 76 |
| d) " | 1000 | 2000 | 1:2,00 | 80,0 | 4,8 | 11 | 52 | 50 | 70 |

* $H_2O$-Gehalt vor Hydrolyse

  bezogen auf einen  durchschnittlichen Wassergehalt des Ausgangsprodukts von 40 %.

## Tabelle III

Allgemeine Arbeitvorschrift III

| | Einsatz (g) A | $H_2O$-Zusatz (g) | Verhältnis A : $H_2O$ | $H_2O$-Gehalt (%) * | $SO_3$ (%) B | NaOH,45% (g) C | Unsulf. (g) D | $S_1$ (%) | $S_2$ (%) |
|---|---|---|---|---|---|---|---|---|---|
| a) Ölsäuremethylester | 300 | 400 | 1:1,33 | 74,3 | 4,8 | 16 | 28 | 50 | 88 |

* $H_2O$-Gehalt vor Hydrolyse

bezogen auf einen durchschnittlichen Wassergehalt des Ausgangsprodukts von 40 %.

## Tabelle IV

| | Einsatz (g) A | $H_2O$-Zusatz (g) | Verhältnis A : $H_2O$ | $H_2O$-Gehalt (%) * | $SO_3$ (%) B | NaOH,45% (g) C | Unsulf. (g) D | $S_1$ (%) | $S_2$ (%) |
|---|---|---|---|---|---|---|---|---|---|
| a) Ölsäuremethylester | 1000 | 500 | 2:1 | 60 | 8,2 | 90 | 108 | 57 | 78 |

* $H_2O$-Gehalt vor Hydrolyse

bezogen auf einen durchschnittlichen Wassergehalt des Ausgangsprodukts von 40 %.

**Patentansprüche**

1. Verfahren zur Herstellung hochsulfatierter Fettsäuren, Hydroxifettsäuren oder oxalkylierter Hydroxifettsäuren, gekennzeichnet durch folgende Schritte:
   - Sulfatierung von ungesättigten Fettsäureestern und/oder gesättigten oder ungesättigten Hydroxifettsäureestern und/oder oxalkylierten gesättigten oder ungesättigten Hydroxifettsäureestern,
   - Neutralisation des Reaktionsgemischs,
   - gegebenenfalls Abtrennung der Salzsole,
   - Verdünnung des neutralen oder alkalisch stabilisierten Estersulfats mit Wasser,
   - Zugabe einer dem organisch gebundenen Schwefelsäuregehalt höchstens äquimolaren Laugenmenge,
   - Hydrolyse der alkalischen Lösung bei Temperaturen zwischen 20°C und 160°C, vorzugsweise 40 - 90°C,
   - Trennung der im wesentlichen aus nicht sulfatiertem Ausgangsprodukt bestehenden Ölphase von der die hochsulfatierten Fettsäuren enthaltenden wässrigen Phase,
   - Isolierung der hochsulfatierten Fettsäuren.

2. Verfahren nach Anspruch 1, <u>dadurch gekennzeichnet</u>, daß zur Verdünnung des neutralisierten, gegebenenfalls entsalzten Estersulfatgemischs mit mindestens der halben Gewichtsmenge Wasser, bezogen auf das Gewicht des Sulfatierungsgemischs, verdünnt wird.

3. Verfahren nach Anspruch 1 oder 2, <u>dadurch gekennzeichnet</u>, daß das neutrale, gegebenenfalls entsalzte Estersulfatgemisch mit der doppelten bis dreifachen Gewichtsmenge Wasser, bezogen auf das Gewicht des Sulfatierungsgemischs, verdünnt wird.

4. Verfahren nach einem der Ansprüche 1 bis 3, <u>dadurch gekennzeichnet</u>, daß der Wassergehalt der alkalischen Mischung zu Beginn der Hydrolyse 60 bis 95 Gew.-% bezogen auf die Gesamtmischung, beträgt.

5. Verfahren nach einem der Ansprüche 1 bis 4, <u>dadurch gekennzeichnet</u>, daß der Wassergehalt der alkalischen Mischung zu Beginn der Hydrolyse 70 bis 90 Gew.-%, bezogen auf die Gesamtmischung, beträgt.

6. Verfahren nach einem der Ansprüche 1 bis 5, <u>dadurch gekennzeichnet</u>, daß das gegebenenfalls entsalzte Estersulfatgemisch mit 10 Gew.-% bis 50 Gew.-% , bezogen auf das nicht hydrolysierte Estersulfat, eines bereits hydrolysierten Estersulfats vermischt und anschließend der weiteren Hydrolyse unterworfen wird.

7. Verfahren nach einem der Ansprüche 1 bis 6, <u>dadurch gekennzeichnet</u>, daß die Hydrolyse des Estersulfats unter Druck erfolgt.

8. Verfahren nach einem der Ansprüche 1 bis 7, <u>dadurch gekennzeichnet</u>, daß es sich bei den eingesetzten Fettsäureestern beziehungsweise Fettsäureesterderivaten um Ester niedrigmolekularer, wasserlöslicher ein- und zweiwertiger Alkohole handelt.

9. Verfahren nach einem der Ansprüche 1 bis 8, <u>dadurch gekennzeichnet</u>, daß die ungesättigten Fettsäureester im Gemisch mit gesättigten Fettsäureestern eingesetzt werden.

10. Verfahren nach einem der Ansprüche 1 bis 9, <u>dadurch gekennzeichnet</u>, daß bei Einsatz eines Gemisches verschiedener Ester in die Sulfatierungsreaktion und/oder die Hydrolyse die Ester bezüglich Fettsäurebasis und/oder Sulfatierungsgrad gleich oder verschieden sein können.

11. Verfahren nach einem der Ansprüche 1 bis 10, <u>dadurch gekennzeichnet</u>, daß die eingesetzten Fettsäureester im wesentlichen frei sind von Fettsäuren.

12. Verfahren nach einem der Ansprüche 1 bis 11, <u>dadurch gekennzeichnet</u>, daß die in der Hydrolysestufe eingesetzten sulfatierten Fettsäureester im wesentlich frei sind von Aufspaltungsprodukten.

13. Verfahren nach einem der Ansprüche 1 bis 12, <u>dadurch gekennzeichnet</u>, daß die Hydrolyse mit starken und/oder schwachen Basen durchgeführt wird.

## Claims

1. Process for the production of highly sulfated fatty acids, hydroxy fatty acids or oxalkylated hydroxy fatty acids characterized by the following steps:
   - sulfatation of unsarurated fatty acids and/or saturated or unsaturated hydroxy fatty acid esters and/ or oxalkylated saturated or unsaturated hydroxy fatty acid esters,
   - neutralization of the reaction mixture,
   - optionally separation of the brine,
   - dilution of the neutral or alkaline stabilized ester sulfate with water,
   - addition of lye the amount of which at maximum is equimolar to the organically bonded sulfuric acid content,
   - hydrolysis of tine alkaline solution at temperatures between 20°C and 160°C, preferably 40 to 90°C,
   - separation of the oil-phase which substantially consists of non-sulfated starting product from the aqueous phase which comprises the highly sulfated fatty acids,
   - isolation of the highly sulfated fatty acids.

2. The process according to claim 1 wherein dilution of the neutralized, optionally desalinated ester sulfate mixture is effected with at least half of the weight amount of water, relative to the weight of the sulfatation mixture.

3. The process according to claims 1 or 2 wherein the neutral, optionally desalinated ester sulfate mixture is diluted with double to triple the weight amount of water, relative to the weight of the sulfatation mixture.

4. The process according to any one of claims 1 to 3 wherein the water content of the alkaline mixture at the beginning of the hydrolysis amounts to 60 to 95%-wt, relative to the total mixture.

5. The process according to any one of claims 1 to 4 wherein the water content of the alkaline mixture at the beginning of the hydrolysis amounts to 70 to 90%-wt, relative to the total mixture.

6. The process according to any one of claims 1 to 5 wherein the optionally desalinated ester sulfate mixture is mixed with 10 to 50%-wt, relative to the nonhydrolyzed ester sulfate, of an already hydrolyzed ester sulfate and subsequently subjected to further hydrolysis.

7. The process according to any one of claims 1 to 6 wherein the hydrolysis of the ester sulfate is carried cut under pressure.

8. The process according to any one of claims 1 to 7 wherein the used fatty acid esters or fatty acid ester derivatives, respectively, are esters of low-molecular, water-soluble, mono- or bivalent alcohols.

9. The process according to any one of claims 1 to 8 wherein the unsaturated fatty acid esters are used in admixture with saturated fatty acid esters.

10. The process according to any one of claims 1 to 9 wherein in case of using a mixture of different esters in the sulfatation reaction and/or in the hydrolysis, the esters may be the same of different with respect to the fatty acid bases and/or sulfatation degree.

11. The process according to any one of claims 1 to 10 wherein the fatty acid esters used are substantially free from fatty acids.

12. The process according to any one of claims 1 to 11 wherein the sulfated fatty acid esters used in the stage of hydrolysis are substantially free from cleavage products.

13. The process according to any one of claims 1 to 12 wherein the hydrolysis is carried out with strong and/ or weak bases.

## Revendications

1. Procédé de préparation d'acides gras hydroxylés et oxalkylés, d'acides gras hydroxylés, ou d'acides gras, fortement sulfatés, caractérisé en ce qu'il comprend les étapes suivantes :

- sulfatation d'acides gras insaturés et/ou d'acides gras hydroxylés, saturés ou insaturés et/ou d'acides gras hydroxylés, saturés ou insaturés, oxalkylés,
- neutralisation du mélange réactionnel,
- éventuellement séparation de la solution de sel,
- dilution de l'ester sulfate neutre ou stabilisé par voie alcaline avec de l'eau,
- addition d'une proportion de lessive au plus équimolaire à la teneur en acide sulfurique organiquement lié,
- hydrolyse de la solution alcaline à des températures comprises entre 20 et 160°C, de préférence 40 et 90°C,
- séparation de l'oléophase essentiellement constituée de produit de départ non sulfaté d'avec la phase aqueuse contenant les acides gras fortement sulfatés,
- isolement des acides gras fortement sulfatés.

2. Procédé suivant la revendication 1, caractérisé en ce que, en vue de la dilution du mélange d'ester sulfate neutralisé, éventuellement dessalé, on dilue avec au moins la demi-proportion pondérale d'eau, par rapport au poids du mélange de sulfatation.

3. Procédé suivant la revendication 1 ou 2, caractérisé en ce que l'on dilue le mélange d'ester sulfate neutre, éventuellement dessalé, avec la proportion pondérale double à triple d'eau, par rapport au poids du mélange de sulfatation.

4. Procédé suivant l'une quelconque des revendications 1 à 3, caractérisé en ce que, au début de l'hydrolyse, la teneur en eau du mélange alcalin varie de 60 à 95% en poids, par rapport au mélange total.

5. Procédé suivant l'une quelconque des revendications 1 à 4, caractérisé en ce que, au début de l'hydrolyse, la teneur en eau du mélange alcalin varie de 70 à 90% en poids, par rapport au mélange total.

6. Procédé suivant l'une quelconque des revendications 1 à 5, caractérisé en ce que l'on mélange le mélange d'ester sulfate éventuellement dessalé à 10% en poids à 50% en poids, par rapport à l'ester sulfate non hydrolysé, d'un ester sulfate déjà hydrolysé et on soumet ensuite le mélange à une hydrolyse plus poussée.

7. Procédé suivant l'une quelconque des revendications 1 à 6, caractérisé en ce que l'hydrolyse de l'ester sulfate s'effectue sous pression.

8. Procédé suivant l'une quelconque des revendications 1 à 7, caractérisé en ce que, dans le cas des dérivés d'esters d'acides gras ou des esters d'acides gras mis en oeuvre, il s'agit d'esters dalcools mono- et dihydroxylés, solubles dans l'eau, de faible poids moléculaire.

9. Procédé suivant l'une quelconque des revendications 1 à 8, caractérisé en ce que l'on met en oeuvre les esters d'acides gras insaturés en mélange à des esters d'acides gras saturés.

10. Procédé selon l'une quelconque des revendications 1 à 9, caractérisé en ce que lors de la mise en oeuvre d'un mélange de divers esters au cours de la réaction de sulfatation et/ou de l'hydrolyse, les esters peuvent être identiques ou différents eu égard à la base des acides gras et/ou au degré de sulfatation.

11. Procédé suivant l'une quelconque des revendications 1 à 10, caractérisé en ce que les esters d'acides gras mis en oeuvre sont essentiellement dépourvus d'acides gras.

12. Procédé suivant l'une quelconque des revendications 1 à 11, caractérisé en ce que les esters d'acides gras sulfatés utilisés au cours de l'étape d'hydrolyse sont essentiellement dépourvus de produits de scission ou de décomposition.

13. Procédé suivant l'une quelconque des revendications 1 à 12, caractérisé en ce que l'on entreprend l'hydrolyse avec des bases fortes et/ou faibles.